# EUROPEAN PATENT APPLICATION

(11) **EP 2 777 620 A1**
(43) Date of publication of application: **17.09.2014**
(21) Application number: 14157156.2
(22) Date of filing: 28.02.2014
(51) Int. Cl.: A61F 2/30, A61F 2/38

(54) **Orthopaedic knee prosthesis**

(30) Priority: 14.03.2013 US 201313804400
(71) Applicant: DePuy (Ireland), Co Cork (IE)
(72) Inventor: Leszko, Filip, Warsaw, Indiana 46581 (US)
(74) Representative: Belcher, Simon James

(57) **Abstract**

A knee prosthesis for use during performance of a knee replacement procedure includes a plurality of stem components in a range of various sizes, a plurality of femoral components in a range of various sizes, and a plurality of tibial trays in a range of various sizes.

Each of the stem components is compatible with each of the femoral components and the tibal trays. Some of the stem components have a single slot formed therein, and other stem components having a pair of slots formed in it.

## Description

The present invention relates generally to an implantable orthopaedic prosthesis, and more particularly to an implantable knee prosthesis.

During the lifetime of a patient, it may be necessary to perform a joint replacement procedure on the patient as a result of, for example, disease or trauma. The joint replacement procedure may involve the use of a prosthesis which is implanted into one or more of the patient's bones. In the case of a knee replacement procedure, a tibial tray is implanted into the patient's tibia. A bearing is secured to the tibial tray. The condyle surfaces of a replacement femoral component bear against the tibial bearing.

Such a knee prosthesis may also include a number of elongated intramedullary stem components which are implanted in the patient's tibia and/or femur. To secure a stem component to the patient's tibia and/or femur, the intramedullary canal of the patient's tibia and/or femur is first surgically prepared (e.g., reamed) such that the stem component may be subsequently implanted therein. In some designs, the stem component is implanted in the patient's bone by use of cementless fixation. One type of such a design is known as a "press fit" stem component.

The invention provides an implantable orthopaedic knee prosthesis assembly which includes a prosthetic knee component configured to be implanted into an end of a long bone that forms a knee joint of a patient. The prosthetic knee component has a tapered bore formed in it. The assembly also includes a first plurality of stem components, with each of the first plurality of stem components having a tapered post configured to be received separately into the tapered bore of the prosthetic knee component and an elongated body extending away from the tapered post and terminating at a distal tip. The elongated body has a number of flutes formed in it. A post taper angle and a post diameter are the same in each of the first plurality of stem components. Each of the first plurality of stem components has a single slot formed in the elongated body that extends from the distal tip toward the tapered post. The assembly further includes a second plurality of stem components, with each of the second plurality of stem components having a tapered post configured to be received separately into the tapered bore of the prosthetic knee component, and an elongated body extending away from the tapered post and terminating at a distal tip. The elongated body has a number of flutes formed in it. The post taper angle and the post diameter of each of the second plurality of stem components are the same as those dimensions of each of the other of the second plurality of stem components and of each of the first plurality of stem components. A pair of slots are formed in the elongated body of each of the second plurality of stem components and extend from the distal tip toward the tapered post. The pair of slots extend perpendicular to one another.

Optionally, each of the second plurality of stem components may have a body diameter that is larger than that of each of the first plurality of stem components.

Optionally, the prosthetic knee component may be a tibial tray which can be implanted into a proximal end of a tibia of the patient. The tibial tray includes a platform with a stem post extending inferiorly from an inferior surface of the platform, with the tapered bore being formed in the stem post.

Optionally, the prosthetic knee component may be a femoral component which can be implanted into a distal end of a femur of the patient. The femoral component includes a bearing surface having a medial condyle surface and a lateral condyle surface, a backside surface opposite the bearing surface, and a stem post extending superiorly away from the backside surface. The tapered bore is formed in the stem post.

Optionally, the prosthetic knee component may be a stem adaptor which can be secured to a tibial tray and implanted into a proximal end of a tibia of the patient. The tapered bore formed is formed in an inferior end of the stem adaptor.

Optionally, the prosthetic knee component may be a femoral sleeve component which can be secured to a femoral component and implanted into a distal end of a femur of the patient. The tapered bore is formed in a superior end of the femoral sleeve component.

Optionally, the prosthetic knee component may be a tibial sleeve component which can be secured to a tibial tray and implanted into a proximal end of a tibia of the patient. The tapered bore is formed in an inferior end of the femoral sleeve component.

Optionally, the longitudinal axis of both the flutes and the single slot of each of the first plurality of stem components extends in the superior/inferior direction.

Optionally, the longitudinal axis of both the flutes and each of the pair of slots of each of the second plurality of stem components extends in the superior/inferior direction.

The invention also provides an implantable orthopaedic knee prosthesis assembly which includes first and second knee stem components configured to be implanted into the intramedullary canal of a long bone that forms a knee joint of a patient. The first knee stem component includes a tapered post configured to be received separately into a tapered bore of a prosthetic knee component, with the tapered post of the first knee stem component having a first post taper angle and a first post diameter. The first knee stem component also includes an elongated body extending away from the tapered post and terminating at a distal tip. The elongated body has formed therein a number of flutes and a single slot extending from the distal tip toward the tapered post. The second knee stem component includes a tapered post configured to be received separately into a tapered bore of a prosthetic knee component, with the tapered post of the second knee stem component having a second post taper angle that is the same as the first post taper angle and a second post diameter that is the same as the first post diameter. The second knee component also has an elongated body extending away from the tapered post and terminating at a distal tip, with the elongated body having formed therein a number of flutes and a pair of slots extending from the distal tip toward the tapered post. The pair of slots are arranged perpendicular to one another.

Optionally, the second knee stem component may have a body diameter that is larger than that of the first knee stem component.

Optionally, both the tapered post of the first knee stem component and the tapered post of the second knee stem component are configured to be received separately into a tapered bore of a tibial tray.

Optionally, both the tapered post of the first knee stem component and the tapered post of the second knee stem component are configured to be received separately into a tapered bore of a femoral component.

Optionally, both the tapered post of the first knee stem component and the tapered post of the second knee stem component are configured to be received separately into a tapered bore of a sleeve component.

Optionally, both the tapered post of the first knee stem component and the tapered post of the second knee stem component are configured to be received separately into a tapered bore of a stem adaptor.

Optionally, the longitudinal axis of both the flutes and the single slot of the first knee stem component extends in the superior/inferior direction.

Optionally, the longitudinal axis of both the flutes and each of the pair of slots of the second knee stem component extends in the superior/inferior direction.

The invention also provides an implantable orthopaedic knee prosthesis assembly which includes a tibial tray configured to be implanted into a proximal end of a tibia of the patient, with the tibial tray including a platform with a stem post extending inferiorly from an inferior surface of the platform. The stem post of the tibial tray has a tapered bore formed in it. The implantable orthopaedic knee prosthesis assembly also includes a femoral component configured to be implanted into a distal end of a femur of the patient, with the femoral component including a bearing surface having a medial condyle surface and a lateral condyle surface, a backside surface opposite the bearing surface, and a stem post extending superiorly away from the backside surface. The stem post of the femoral component has a tapered bore formed in it. The implantable orthopaedic knee prosthesis assembly also includes a first plurality of stem components, each of which has a tapered post configured to be received separately into the tapered bore of the tibial tray and the tapered bore of the femoral component and an elongated body extending away from the tapered post and terminating at a distal tip. The elongated body has a number of flutes formed in it. Each of the first plurality of stem components also has a post taper angle and a post diameter that are the same as each of the other of the first plurality of stem components, and a single slot formed in the elongated body and extending from the distal tip toward the tapered post. The implantable orthopaedic knee prosthesis assembly further includes a second plurality of stem components, each of which has a tapered post configured to be received separately into the tapered bore of the tibial tray and the tapered bore of the femoral component, and an elongated body extending away from the tapered post and terminating at a distal tip. The elongated body has a number of flutes formed in it. Each of the second plurality of stem components also has a post taper angle and a post diameter that are the same as each of the other of the second plurality of stem components and the first plurality of stem components, and a pair of slots formed in the elongated body and extending from the distal tip toward the tapered post. The pair of slots is arranged perpendicular to one another.

Optionally, the second plurality of stem components may have a body diameter that is larger than each of the first plurality of stem components.

Optionally, the longitudinal axis of both the flutes and the single slot of each of the first plurality of stem components extends in the superior/inferior direction.

Optionally, the longitudinal axis of both the flutes and each of the pair of slots of each of the second plurality of stem components extends in the superior/inferior direction.

Optionally, the implantable orthopaedic knee prosthesis assembly may also include a stem adaptor configured to be secured to the tibial tray and implanted into the proximal end of the tibia of the patient. A tapered bore is formed in an inferior end of the stem adaptor, with the tapered post of each of the first plurality of stem components and the second plurality of stem components is configured to be received separately into the tapered bore of the stem adaptor.

Optionally, the implantable orthopaedic knee prosthesis assembly may also include a femoral sleeve component configured to be secured to the femoral component and implanted into the distal end of the femur of the patient. A tapered bore is formed in a superior end of the femoral sleeve component, with the tapered post of each of the first plurality of stem components and the second plurality of stem components is configured to be received separately into the tapered bore of the femoral sleeve component.

Optionally, the implantable orthopaedic knee prosthesis assembly may also include a tibial sleeve component configured to be secured to the tibial tray and implanted into the proximal end of the tibia of the patient. A tapered bore is formed in an inferior end of the tibial sleeve component, with the tapered post of each of the first plurality of stem components and the second plurality of stem components is configured to be received separately into the tapered bore of the tibial sleeve component.

The invention is described below by way of example with reference to the accompanying drawings, in which:
FIG. 1 is an exploded perspective view of an orthopaedic knee prosthesis, in which a portion of the stem post of the tibial tray has been cut away for clarity.
FIG. 2 is a perspective view showing a stem component taper locked to both the tibial tray and the femoral component of the orthopaedic knee prosthesis of FIG. 1, in which a pair of optional sleeve components and a sleeve adaptor are shown exploded from the assembled knee prosthesis, with a portion of the stem adaptor being cut away.
FIG. 3 is a diagrammatic elevation view showing a number of stem components with differing body diameters.
FIG. 4 is a diagrammatic cross sectional view taken along line 4-4 of FIG. 3, as viewed in the direction of the arrows.

Terms representing anatomical references, such as anterior, posterior, medial, lateral, superior and inferior, may be used throughout this document to refer to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

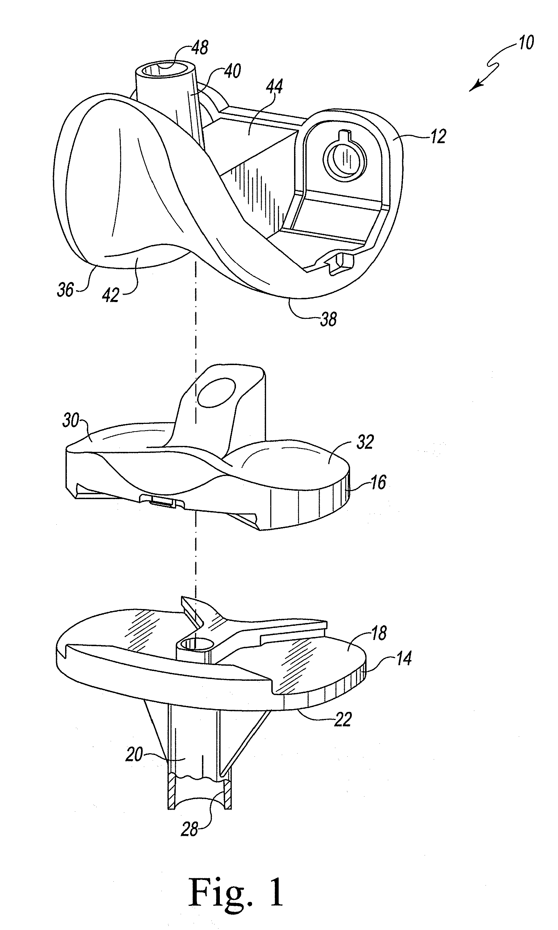

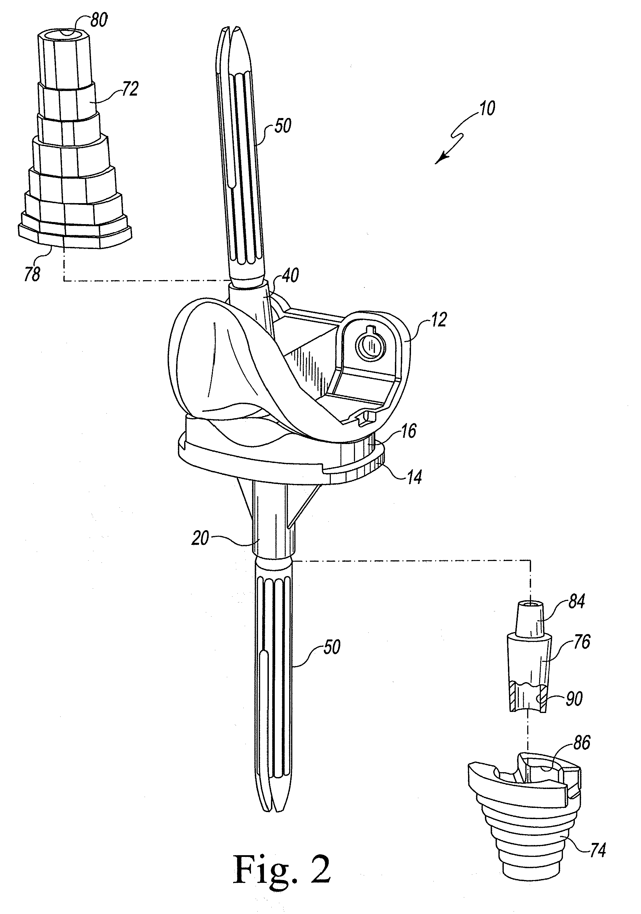

Referring to the drawings, FIG. 1 shows an implantable knee prosthesis 10 for use in the performance of an orthopaedic knee replacement procedure. The knee prosthesis 10 includes a femoral component 12, a tibial tray 14, and a bearing 16. As can be seen in FIGS. 2 to 4, the knee prosthesis 10 also includes a number of stem components 50. As will be discussed below, each of the variously-sized stem components 50 may be secured to either the tibial tray 14 or the femoral component 12.

The tibial tray 14 is configured to be implanted into a surgically-prepared end of a patient's proximal tibia (not shown). The tibial tray 14 includes a platform 18 having an elongated stem post 20 extending inferiorly away from its inferior surface 22. The elongated tibial stem post 20 is configured to receive the stem components 50. As can be seen in FIG. 1, the stem post 20 of the tibial tray 14 has a tapered bore 28 formed therein into which a tapered post 24 of one of the stem components 50 may be advanced to taper lock the post 24 (and hence the stem component 50) and the tibial tray 14 to one another (as shown in FIG. 2). In this way, the stem component 50 may then be implanted into a surgically-prepared (e.g., reamed or broached) intramedullary canal of the patient's tibia.

The bearing 16 is securable to the tibial tray 14. In particular, the bearing 16 may be snap-fit to the tibial tray 14. In this way, the bearing 16 is fixed relative to the tibial tray 14 (i.e., it is not rotatable or moveable in the anterior/posterior or medial/lateral directions). It is envisaged that the tibial tray may be secured in a manner that allows it to rotate relative to the tibial tray 14 in other devices.

The bearing 16 has a lateral bearing surface 30 and a medial bearing surface 32. The bearing surfaces 30, 32 are configured to articulate with a lateral condyle surface 36 and a medial condyle surface 38, respectively, of the femoral component 12. Specifically, the femoral component 12 is configured to be implanted into a surgically-prepared distal end of the patient's femur (not shown), and is configured similarly to the patient's natural femoral condyles. As such, the lateral condyle surface 36 and the medial condyle surface 38 are configured (e.g., curved) in a manner which is similar the condyles of the natural femur. The lateral condyle surface 36 and the medial condyle surface 38 are spaced apart from one another so as to define an intercondylar notch between them.

The condyle surfaces 36, 38 are formed in a bearing surface 42 of the femoral component 12. The femoral component 12 also includes an elongated stem post 40, extending superiorly away from its opposite backside surface 44. The elongated femoral stem post 40 is configured to receive the stem components 50. Specifically, as can be seen in FIG. 1, the femoral component 12 has a tapered bore 48 formed therein into which a tapered post 24 of one of the stem components 50 may be advanced to taper lock the post 24 (and hence the stem component 50) and the femoral component 12 to one another (as shown in FIG. 2). In this way, the stem component 50 may then be implanted into a surgically-prepared (e.g., reamed or broached) intramedullary canal of the patient's femur.

As shown in FIG. 2, the knee prosthesis 10 may also include a number of optional components such as a femoral sleeve component 72, a tibial sleeve component 74, and a sleeve adaptor 76. The sleeve components 72, 74 may be used to facilitate implantation of the femoral component 12 and the tibial tray 14, respectively, in the presence of reduced bone quality in the patient's femur or tibia. The femoral sleeve component 72 is configured to be secured to the femoral component 12 so as to be positioned between the femoral component 12 and the stem component 50. In particular, the inferior end 78 of the femoral sleeve component 72 has a bore (not shown) formed in it that may be taper locked to the outer surface of the femoral component's stem post 40 to lock the sleeve component 72 to the femoral component 12. The opposite, superior end of the femoral sleeve component 72 is configured to receive the stem components 50. Specifically, the superior end of the femoral sleeve component 72 has a tapered bore 80 formed therein into which a tapered post 24 of one of the stem components 50 may be advanced to taper lock the post 24 (and hence the stem component 50) and the femoral sleeve component 72 to one another.

The tibial sleeve component 74 may be similarly configured, with a bore formed in its superior end which is taper locked to the stem post 20 of the tibial tray 14, and with its opposite, inferior end having a tapered bore formed in it into which a tapered post 24 of one of the stem components 50 may be advanced to taper lock the post 24 (and hence the stem component 50) and the tibial sleeve component 74 to one another.

Alternatively, as shown in FIG. 2, the tibial sleeve component 74 may be used in conjunction with the stem adaptor 76. In such a device, the stem adaptor 76 is used to secure both the stem components 50 and the tibial sleeve component 74 to the tibial tray 14. In particular, the stem adaptor 76 includes a tapered post 84 that is identical in shape and size to the tapered post 24 of each of the stem components 50. As such, the tapered post 84 of the stem adaptor 76 may be advanced into the tapered bore 28 formed in the tibial tray's stem post 20 to taper lock the post 84 (and hence the stem adaptor 76) and the tibial tray 14 to one another. The tibial sleeve component 74 is configured to be secured to the stem adaptor 76 so as to be positioned between the tibial tray 14 and the stem component 50. In particular, the tibial sleeve component 74 has a bore 86 formed in it that extends through its entire length and hence is open to both its superior end and its inferior end. The tibial sleeve component 74 may be advanced over the stem adaptor 76 such that the tapered sidewalls forming the bore 86 of the tibial sleeve component 74 engage to the tapered outer surface of the stem adaptor 76 to taper lock the sleeve component 74 to the stem adaptor 76 to one another. As can be seen in FIG. 2, the inferior end of the stem adaptor 76 is configured to receive the stem components 50. Specifically, the inferior end of the stem adaptor 76 has a tapered bore 90 formed therein into which a tapered post 24 of one of the stem components 50 may be advanced to taper lock the post 24 (and hence the stem component 50) and the stem adaptor 76 to one another.

The components of the knee prosthesis 10 that engage the natural bone, such as the femoral component 12, the tibial tray 14, the stem components 50, and the sleeve components 72, 74, along with the stem adaptor 76, may be constructed with an implant-grade biocompatible metal, although other materials may also be used. Examples of such metals include cobalt, including cobalt alloys such as a cobalt chromium alloy, titanium and including titanium alloys such as a Ti6A14V alloy, and certain stainless steels. Such metallic components may also be coated with a surface treatment, such as hydroxyapatite, to enhance biocompatibility. Moreover, the surfaces of the metallic components that engage the natural bone may be textured to facilitate securing the components to the bone. Such surfaces may also be porous coated to promote bone ingrowth for permanent fixation.

The bearing 16 may be constructed with a material that allows for smooth articulation between the bearing 16 and the femoral component 12, such as a polymeric material. A suitable polymeric material is a polyethylene especially an ultrahigh molecular weight polyethylene (UHMWPE).

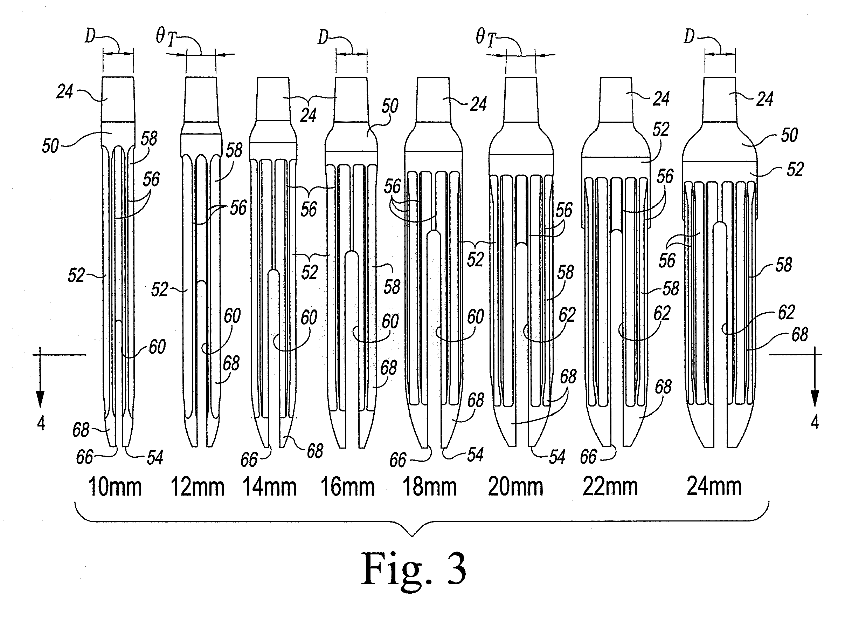

As can be seen in FIG. 3, each of the stem components 50 has an elongated, generally cylindrical stem body 52. The tapered post 24 is positioned at a proximal end of the elongated stem body 52. The elongated stem body 52 extends distally away from the tapered post 24 and terminates at rounded distal end 54 that defines the inferior-most surface of the stem component 50 when it is secured to a tibial tray 14 or the superior-most surface of the stem component 50 when it is secured to a femoral component 12.

As can be seen in FIG. 3, a number of elongated flutes 56 are formed in the outer annularly-shaped surface 58 of the stem body 52. As can also be seen in FIG. 3, the longitudinal axis of each of the flutes 56 is parallel to the longitudinal axis of the stem component 50 and hence is arranged in the superior/inferior direction.

The stem component 50 may be provided in a number of different configurations in order to fit the needs of a given patient's anatomy. In particular, the stem component 50 may be configured in various different lengths to conform to the patient's anatomy (e.g., a relatively long stem component 50 for use with a long femur or tibia, a relatively short stem for use with a short femur or tibia, etcetera). For example, the stem component 50 may be provided in three different lengths (e.g., 60 mm, 110 mm, and 160 mm).

The stem component 50 may also be provided in varying body diameters to fit the needs of a given patient's anatomy. The body diameter of a given stem component 50 is the stem component's medial/lateral cross sectional width in the cylindrical midsection of the stem component's body 52 (i.e., not at its tapered post 24 or its distal tip 54). For example, the stem component 50 may be provided in eight different diameters (e.g., 10 mm, 12 mm, 14 mm, 16 mm, 18 mm, 20 mm, 22 mm, and 24 mm) at each of the three different lengths (e.g., 60 mm, 110 mm, and 160 mm). In other words, in such a device, 24 differently-sized stem components 50 may be provided in eight different diameters and three different lengths.

Likewise, the femoral component 12, the tibial tray 14, and the sleeve components 72, 74 may be provided in various different sizes to fit the needs of a given patient's anatomy. However, each of the differently-sized stem components 50 is compatible with each of the differently-sized femoral components 12, tibial trays 14, and sleeve components 72, 74, along with the stem adaptor 76. In particular, as described above, the tibial tray 12 has a tapered bore 28 formed in its stem post 20, the femoral component 12 has a tapered bore 48 formed in its stem post 40, the femoral sleeve component 72 has a tapered bore 80 formed in its superior end, the stem adaptor 76 has a tapered bore 90 formed in its inferior end and, optionally, the tibial sleeve component 74 may have a tapered bore (not shown) formed in its inferior end (when the tibial sleeve component 74 is used without the stem adaptor 76). The tapered post 24 of a given stem component 50 may be advanced into any of such tapered bores 28, 48, 80, 90 to taper lock the tapered post 24 (and hence the stem component 50) and the tibial tray 14 or the femoral component 12 or the sleeve component 72, 74 or the stem adaptor 76 to one another. The geometry of the tapered bores 28, 48, 80, 90 of each of the differently-sized tibial trays 14, the differently-sized femoral components 12, the differently-sized sleeve components 72, 74, and the stem adaptor 76 is identical. Specifically, each of the tapered bores 28, 48, 80, 90 has a taper angle and a diameter that are the same as the taper angle and diameter of the other tapered bores.

Likewise, the geometries of the tapered posts 24 of each of the differently-sized stem components 50 are identical. In particular, as shown in FIG. 3, the post 24 of each of the differently-sized stem components 50 has a diameter (D) that is the same the diameters of the posts of the other stem components. As shown in FIG. 3, the taper angle (θ_{T}) of each stem component's post 24 is the same across the range of sizes of the stem components 50. Because the taper features of the tibial trays 14, femoral components 14, sleeve components 72, 74, and stem components 50 are commonly sized across the range of component sizes, each of the differently-sized stem components 50 is compatible with each of the differently-sized femoral components 12, each of the differently-sized tibial trays 14, and each of the differently-sized sleeve components 72, 74. As noted above, the tapered post 84 of the stem adaptor 76 is identical in size and shape to the tapered post 24 of the stem components 50 and its tapered bore 90 is identical in shape and size to the tapered bore 28 of the tibial tray 14. The stem adaptor 76 is therefore compatible with each of the differently-sized tibial trays 14 and each of the differently-sized stem components 50.

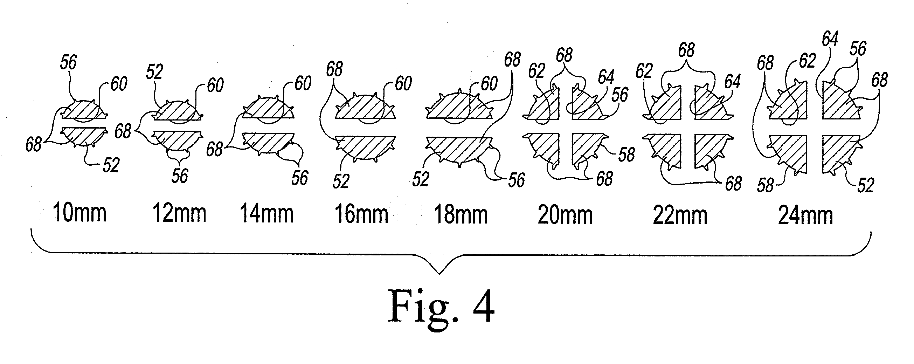

As shown in FIGS. 3 and 4, in contrast to prior designs in which all of the differently-sized stem components across a range of component sizes share a common slot configuration, some of the stem components 50 across the range of component sizes have a single elongated slot 60 formed therein, with others of the stem components having a pair of elongated slots 62, 64 formed therein. In particular, in the device shown in the drawings, the five smallest-diameter stem components 50 (e.g., 10 mm, 12 mm, 14 mm, 16 mm, and 18 mm) have a single slot 60, with the three largest-diameter stem components 50 (e.g., 20 mm, 22 mm, and 24 mm) having the pair of elongated slots 62, 64.

As can be seen in FIG. 4, in the stem components 50 having two slots formed therein, the slots 62, 64 are arranged in a perpendicular arrangement relative to one another. In other words, when viewed in a medial/lateral cross section through the portion of the stem body 52 having the slots 62, 64 (such as the cross section views of FIG. 4), the slot 62 extends perpendicularly to the slot 64 so as to divide the stem body 52 into four equal sections.

As can be seen in FIGS. 3 and 4, the longitudinal axis of each of the elongated slots 60, 62, 64 of the stem components 50 is parallel to the longitudinal axis of each of the flutes 56 and, hence, also parallel to the longitudinal axis of the stem component 50 itself. A distal end 66 of the slots 60, 62, 64 is defined in (i.e., opens into) the distal tip 54. The slots 60, 62, 64 extend from their distal ends 66 in the direction toward the tapered post 24 and terminate in the stem body 52 at a location between the tapered post 24 and the distal end 66. As such, the slots 60, 62, 64 create arcuate-shaped cantilevered flanges 68. As can be seen in FIG. 4, in the case of the stem components 50 with only a single slot 60, two of such cantilevered flanges 68 are formed in the stem body 52, whereas four of such cantilevered flanges 68 are formed in the stem components 50 having a pair of slots 62, 64. The slot 60 is centred on the stem body 52 such that the two cantilevered flanges 68 formed in the stem components 50 having only a single slot 60 are of equal size. The slots 62, 64 are likewise centred on the stem body 52 and, as noted above, arranged in a perpendicular arrangement relative to one another. As such, the four cantilevered flanges 68 formed in the stem components 50 having a pair of slots 62, 64 are likewise of equal size.

As described above, the stem components 50 have a constant post diameter (D) and post taper angle (θ_{T}) across their range of sizes, but the number of elongated slots formed in the stem body 52 varies across their range of sizes with some stem components 50 having a single slot 60 and others having a pair of slots 62, 64 perpendicularly arranged with one another. As a result, an enhanced stem component set is provided that balances parameters such as, amongst others, net closure force of the stem's cantilevered flanges 68, maximum principal stress, flute engagement area, slot width, and slot diameter across the range of sizes.

While the stem components 50 described above with reference to the drawings are straight press-fit stem components, other types of press-fit stems are also contemplated including bowed and offset press-fit stem components.

## Claims

1. An implantable orthopaedic knee prosthesis assembly, comprising:
a prosthetic knee component configured to be implanted into an end of a long bone that forms a knee joint of a patient, the prosthetic knee component having a tapered bore formed therein, and
a first plurality of stem components, each of the first plurality of stem components having (i) a tapered post configured to be received separately into the tapered bore of the prosthetic knee component, (ii) an elongated body extending away from the tapered post and terminating at a distal tip, the elongated body having a number of flutes formed therein, (iii) a post taper angle and a post diameter that are the same as each of the other of the first plurality of stem components, and (iv) a single slot formed in the elongated body and extending from the distal tip toward the tapered post, and
a second plurality of stem components, each of the second plurality of stem components having (i) a tapered post which can be received separately into the tapered bore of the prosthetic knee component, (ii) an elongated body extending away from the tapered post and terminating at a distal tip, the elongated body having a number of flutes formed therein, (iii) a post taper angle and a post diameter that are the same as the post taper angle and the post diameter of each of the other of the second plurality of stem components and of each of the first plurality of stem components, and (iv) a pair of slots formed in the elongated body and extending from the distal tip toward the tapered post, the pair of slots being arranged perpendicular to one another.

2. The implantable orthopaedic knee prosthesis assembly of claim 1, in which each of the second plurality of stem components has a body diameter that is larger than that of each of the first plurality of stem components.

3. The implantable orthopaedic knee prosthesis assembly of claim 1, in which: the prosthetic knee component comprises a tibial tray configured to be implanted into a proximal end of a tibia of the patient, the tibial tray includes a platform with a stem post extending inferiorly from an inferior surface of the platform, and the tapered bore is formed in the stem post.

4. The implantable orthopaedic knee prosthesis assembly of claim 1, in which:
the prosthetic knee component comprises a femoral component configured to be implanted into a distal end of a femur of the patient,
the femoral component includes (i) a bearing surface comprising a medial condyle surface and a lateral condyle surface, and (ii) a backside surface opposite the bearing surface, and (iii) a stem post extending superiorly away from the backside surface, and
the tapered bore is formed in the stem post.

5. The implantable orthopaedic knee prosthesis assembly of claim 1, in which the prosthetic knee component comprises a stem adaptor configured to be secured to a tibial tray and implanted into a proximal end of a tibia of the patient, and the tapered bore is formed in an inferior end of the stem adaptor.

6. The implantable orthopaedic knee prosthesis assembly of claim 1, in which the prosthetic knee component comprises a femoral sleeve component configured to be secured to a femoral component and implanted into a distal end of a femur of the patient, and the tapered bore is formed in a superior end of the femoral sleeve component.

7. The implantable orthopaedic knee prosthesis assembly of claim 1, in which the prosthetic knee component comprises a tibial sleeve component configured to be secured to a tibial tray and implanted into a proximal end of a tibia of the patient, and the tapered bore is formed in an inferior end of the tibial sleeve component.

8. The implantable orthopaedic knee prosthesis assembly of claim 1, in which the longitudinal axis of both the flutes and the single slot of each of the first plurality of stem components extends in the superior/inferior direction.

9. The implantable orthopaedic knee prosthesis assembly of claim 1, in which the longitudinal axis of both the flutes and each of the pair of slots of each of the second plurality of stem components extends in the superior/inferior direction.

10. An implantable orthopaedic knee prosthesis assembly, comprising:
a first knee stem component configured to be implanted into the intramedullary canal of a long bone that forms a knee joint of a patient, the first knee stem component comprising (i) a tapered post configured to be received separately into a tapered bore of a prosthetic knee component, the tapered post of the first knee stem component having a first post taper angle and a first post diameter, and (ii) an elongated body extending away from the tapered post and terminating at a distal tip, the elongated body having formed therein a number of flutes and a single slot extending from the distal tip toward the tapered post, and
a second knee stem component configured to be implanted into the intramedullary canal of a long bone that forms a knee joint of a patient, the second knee stem component comprising (i) a tapered post configured to be received separately into a tapered bore of a prosthetic knee component, the tapered post of the second knee stem component having a second post taper angle that is the same as the first post taper angle and a second post diameter that is the same as the first post diameter, and (ii) an elongated body extending away from the tapered post and terminating at a distal tip, the elongated body having formed therein a number of flutes and a pair of slots extending from the distal tip toward the tapered post, the pair of slots being arranged perpendicular to one another.

11. The implantable orthopaedic knee prosthesis assembly of claim 10, in which the second knee stem component has a body diameter that is larger than the first knee stem component.

12. The implantable orthopaedic knee prosthesis assembly of claim 10, in which both the tapered post of the first knee stem component and the tapered post of the second knee stem component are configured to be received separately into at least one of:
a tapered bore of a tibial tray,
a femoral component,
a sleeve component, and
a stem adaptor.

13. The implantable orthopaedic knee prosthesis assembly of claim 10, in which the longitudinal axis of both the flutes and the single slot of the first knee stem component extends in the superior/inferior direction.

14. The implantable orthopaedic knee prosthesis assembly of claim 10, in which the longitudinal axis of both the flutes and each of the pair of slots of the second knee stem component extends in the superior/inferior direction.

15. An implantable orthopaedic knee prosthesis assembly, comprising:
a tibial tray configured to be implanted into a proximal end of a tibia of the patient, the tibial tray comprising a platform with a stem post extending inferiorly from an inferior surface of the platform, the stem post having a tapered bore formed therein,
a femoral component configured to be implanted into a distal end of a femur of the patient, the femoral component comprising (i) a bearing surface having a medial condyle surface and a lateral condyle surface, (ii) a backside surface opposite the bearing surface, and (iii) a stem post extending superiorly away from the backside surface, the stem post having a tapered bore formed therein,
a first plurality of stem components, each of the first plurality of stem components having (i) a tapered post configured to be received separately into the tapered bore of the tibial tray and the tapered bore of the femoral component, (ii) an elongated body extending away from the tapered post and terminating at a distal tip, the elongated body having a number of flutes formed therein, (iii) a post taper angle and a post diameter that are the same as each of the other of the first plurality of stem components, and (iv) a single slot formed in the elongated body and extending from the distal tip toward the tapered post, and
a second plurality of stem components, each of the second plurality of stem components having (i) a tapered post configured to be received separately into the tapered bore of the tibial tray and the tapered bore of the femoral component, (ii) an elongated body extending away from the tapered post and terminating at a distal tip, the elongated body having a number of flutes formed therein, (iii) a post taper angle and a post diameter that are the same as each of the other of the second plurality of stem components and the first plurality of stem components, and (iv) a pair of slots formed in the elongated body and extending from the distal tip toward the tapered post, the pair of slots being arranged perpendicular to one another.
